# EUROPÄISCHE PATENTSCHRIFT

(11) **EP 2 398 427 B1**
(45) Veröffentlichungstag und Bekanntmachung des Hinweises auf die Patenterteilung: **08.04.2015**
(21) Anmeldenummer: 10705287.0
(22) Anmeldetag: 15.02.2010
(51) Int. Cl.: A61F 5/01

(54) **ORTHESEN-BESCHLAG MIT EINEM KNIEFÜHRUNGSGELENK**
ORTHOTIC FITTING HAVING A KNEE GUIDE JOINT
ARMATURE D'ORTHÈSE PRÉSENTANT UNE ARTICULATION DE GUIDAGE DE GENOU

(30) Priorität: 17.02.2009 DE 102009009250
(43) Veröffentlichungstag der Anmeldung: 28.12.2011
(73) Patentinhaber: Spörer AG, 85049 Ingolstadt (DE)
(72) Erfinder: HARNACK, Roland, 83313 Eisenärtz (DE)
(74) Vertreter: Liebl, Thomas
(86) Internationale Anmeldenummer: PCT/EP2010/000915
(87) Internationale Veröffentlichungsnummer: WO 2010/094444

(56) Entgegenhaltungen:
- EP-A1- 0 267 999
- EP-A1- 0 546 331
- DE-A1-102004 038 191
- US-A- 4 821 707
- US-A- 5 022 391

## Beschreibung

Die Erfindung betrifft einen Orthesen-Beschlag mit einem Knieführungsgelenk nach dem Oberbegriff des Anspruchs 1. Mit einem solchen Orthesen-Beschlag sind unilateral oder mit gegenüberliegenden Beschlägen bilateral ein Unterschenkel-Fassungsteil und ein Oberschenkel-Fassungsteil der Orthese gelenkig als Kniegelenkstütze miteinander verbindbar. Dazu weist ein solcher Beschlag zwei jeweils mit dem Unterschenkel-Fassungsteil und dem Oberschenkel-Fassungsteil fest verbindbare Schenkelteile auf, deren aufeinander zugerichtete Schenkelenden Bestandteile des Knieführungsgelenks sind. Unter den Begriffen Unterschenkel-Fassungsteil und Oberschenkel-Fassungsteil werden allgemein alle Vorrichtungen wie Schalenteile, Gurtelemente, Manschetten, Bandagen, etc. verstanden mit deren Hilfe ein Orthesen-Beschlag mit seinen beiden Schenkelteilen am Unterschenkel und Oberschenkel eines Patienten in einer vorbestimmten Lage festgelegt und fixiert werden kann.

Bei solchen allgemein bekannten Knieorthesen sind die Knieführungsgelenke der Orthesen-Beschläge meistens als einfache Drehgelenke ausgeführt. Der Schwenkbereich ist gegebenenfalls durch Anschläge festgelegt und begrenzt. Beispielsweise werden solche Knieorthesen nach Kreuzbandoperationen angelegt, wodurch die Relativbewegung zwischen Unterschenkel und Oberschenkel nur im Rahmen der Bewegungskinematik der Knieorthese in Verbindung mit einer Stabilisierung des Knies möglich ist.

Die Schwenkgeometrie des menschlichen Knies entspricht nicht einem Drehgelenk sondern einem Gleit-Rollgelenk, bei dem sich die Schwenkachse in Abhängigkeit des Schwenkwinkels im Raum verlagert. Die dem Knie durch die Knieorthese über die Drehgelenke der Orthesen-Beschläge aufgezwungene Schwenkbewegung entspricht damit nicht der Knieschwenkbewegung, so dass auf das Knie, welches durch die Knieorthese entlastet sein soll, in bestimmten Schwenkbereichen ungünstig Zwangskräfte ausgeübt werden. Diese Zwangskräfte können in an sich bekannter Weise etwas reduziert werden, wenn die Orthesen-Drehgelenke etwas oberhalb des Kniegelenks positioniert werden, jedoch ergeben sich auch bei dieser Anordnung noch ungünstige Zwangskräfte auf das Kniegelenk.

Um hier eine Verbesserung zu schaffen sind daher bereits sogenannte polyzentrische Knieorthesen (DE 199 33 197 B4) mit einer Gelenkeinrichtung bekannt, die zwei voneinander beabstandete Schwenkachsen für die schwenkbare Lagerung des distalen Schenkels und des proximalen Schenkels eines Orthesen-Beschlags aufweisen. Zudem greifen die Schenkelenden jeweils über eine Verzahnung ineinander und es sind verstellbare Anschläge für eine Begrenzung des Schwenkbereichs vorgesehen. Derartige zweiachsige Knieorthesen sollen mit ihrer Schwenkgeometrie den natürlichen Bewegungsablauf beim Abbiegen des Kniegelenks besser nachahmen als die weiter oben angegebenen Knieorthesen mit nur einachsigen Orthesen-Beschlägen. Zum einen treten jedoch auch hierbei Zwangskräfte auf das Kniegelenk auf, da auch hier die natürliche Bewegungsnachahmung unvollkommen ist und andererseits ist der Orthesen-Beschlag mit einer Gelenk-Verzahnung aufwendig und teuer.

Weiter ist es bekannt ein gattungsgemäßes Knieführungsgelenk als Vier-Gelenk-Anordnung mit vier achsparallelen Gelenken auszubilden (US 4 821 707 A; US 5 022 391 A). Dazu sind an den Schenkelenden der Beschlagschenkel jeweils Winkelhebelteile mit jeweils zwei beabstandeten Schwenklagern ausgebildet. Dabei ist jeweils das schenkelnähere Schwenklager eines Winkelhebelteils mit den schenkelferneren Schwenklager des anderen Winkelhebelteils durch ein Schwenkelement verbunden, so dass zusätzlich zu den fest an den beiden Beschlagschenkeln angebrachten Winkelhebelteilen zwei in der vorstehenden Weise angelenkte Schwenkelemente verwendet sind.

Aufgabe der Erfindung ist es, einen Orthesen-Beschlag mit einem Knieführungsgelenk vorzuschlagen, dessen Kinematik weitestgehend dem natürlichen Bewegungsablauf eines Kniegelenks entspricht und das zudem einen stabilen, funktionalen und kostengünstigen Aufbau ermöglicht.

Diese Aufgabe wird mit den Merkmalen des Anspruchs 1 gelöst.

Gemäß Anspruch 1 entspricht an einem ersten Winkelhebelteil eines ersten, vorzugsweise unteren, distalen Schenkelteils der Abstand (a) der dortigen beiden Schwenklager dem Abstand (b) der beiden Schwenklager zwischen dem schenkelfernen Gelenklager am ersten Winkelhebelteil und dem schenkelnahen Schwenklager des zweiten Winkelhebelteils des zweiten, vorzugsweise oberen, proximalen Schenkelteils entsprechend dem Abstand der zugeordneten Schwenklager an einem ersten Schwenkelement. Zudem entspricht der Abstand (a) der beiden Schwenklager am ersten Winkelhebel ebenso wie der vorgenannten Abstand (b) dem Abstand (c) der beiden Schwenklager zwischen dem schenkelfernen Schwenklager am zweiten Winkelhebelteil und dem schenkelnahen Schwenklager am ersten Winkelhebelteil und damit dem entsprechenden Abstand der Schwenklager am zweiten Schwenkelement. Während die vorgenannten Abstände gleich groß sind, entspricht jedoch der Abstand (d) der beiden Schwenklager am zweiten Winkelhebelteil nur der Hälfte des Abstands (a) der beiden Schwenklager am ersten Winkelhebelteil. Die hier gemachten Angaben über drei gleiche Abstände und eine halbe Abstandsgröße ergeben in Verbindung mit der Vier-Gelenk-Anordnung eine weitgehend genaue Nachahmung des Bewegungsablaufs eines natürlichen Kniegelenks, wobei kleinere Toleranzen bei den angegebenen Abstandsverhältnissen noch zu guten Ergebnissen führen können.

Eine weitere Anpassungsoptimierung kann nach Anspruch 2 dadurch erreicht werden, dass die Verbindungslinie zwischen den Schwenklagern am zweiten Winkelhebelteil senkrecht zur Schenkelrichtung des zweiten Schenkelteils verläuft, jedoch die Verbindungslinie zwischen den Schwenklagern am ersten Winkelhebelteil in einem Winkel größer 90°, vorzugsweise von ca. 105° zur Schenkelrichtung des ersten Schenkelteils verläuft. Das erste Schenkelteil kann dabei nach Bedarf in einem Abstand vom Knieführungsgelenk auch abgebogen verlaufen.

Mit den in Anspruch 3 angegebenen Abstandsgrößen für die Abstände a, b, c von 22mm bis 30mm, vorzugsweise 26mm und entsprechend einer Abstandsgröße d von 11 mm bis 15mm, vorzugsweise 13mm ist die Bewegungsanpassung an ein natürliches Kniegelenk besonders optimal möglich.

Für eine gewichtsgünstige Ausführung bei hoher Stabilität sollen gemäß Anspruch 4 die Schenkelteile einschließlich der Winkelhebelteile aus einem flachen Material, vorzugsweise aus einem Edelstahlmaterial hergestellt sein. Zudem sollen die Schwenkelemente als Scheibenteile ausgeführt sein, wobei diese gegenüberliegend an den flachen Winkelhebelteilen anliegen, so dass dadurch mit wenig Aufwand eine gute Längsführung in allen Schwenkstellungen erhalten wird.

In einer Weiterbildung nach Anspruch 5 sind diese Scheibenteile zweilagig ausgebildet mit einer stabilen Außenseite aus einer Edelstahllage und einer entsprechenden Innenseite aus einer Messinglage, wobei die beiden Lagen miteinander verklebt sind. Über die Messinglage ist die bei der Schwenkbewegung verschiebbare Anlageverbindung hergestellt, wobei für eine leicht gängige Verschwenkung die Messinglage vorteilhaft als selbstschmierende Schicht wirkt.

Mit den Merkmalen nach Anspruch 6 wird eine einfache konkrete Ausbildung der Schwenklager angegeben. So sind an den Winkelhebelteilen abstehende Lagerbuchsen angebracht, die formschlüssig durch die zugeordneten Bohrungen in den Scheibenteilen gesteckt sind, wobei die Lagerbuchsenlängen den Scheibenteilendicken mit geringem Übermaß entsprechen. In den Lagerbuchsen sind axiale Schraubenbohrungen enthalten zur Aufnahme von Schrauben mit Schraubenköpfen, die Bohrungen der Scheibenteile überdecken und an den Scheibenteilen von außen her anliegen. Damit sind leichtgängige wartungsfreie Schwenklager in Verbindung mit einer einfachen Montage des Knieführungsgelenks realisierbar.

Mit Anspruch 7 wird vorgeschlagen an wenigstens einem der Schenkelteile und an einem relativ dazu beweglichen Schwenkelement, insbesondere an einem Scheibenteil zugeordnete, gegebenenfalls lösbare und versetzbare Anschläge anzubringen für eine vorbestimmte Begrenzung der Schenkel-Schwenkbewegung.

Konkret kann dazu nach Anspruch 8 am ersten Schenkelteil seitlich ein Anschlag so angebracht sein, dass bei einer Schenkelverschwenkung in eine gestrecktere Lage der Anschlag randseitig am ersten Scheibenteil entlang bis zu einem dortigen Anschlag bewegbar ist.

Für eine verbesserte Längsführung kann in einer weiteren Ausgestaltung nach Anspruch 9 am ersten Schenkelteil seitlich eine kanalförmige Führungsnut, gegebenenfalls in Verbindung mit einem Anschlag angebracht sein, in der bei einer Schenkelverschwenkung ein Scheibenrand des ersten Scheibenteils geführt wird. Dabei kann nach Anspruch 10 die zur Scheibenteilfläche hingerichtete und dort anliegende Führungswandfläche zweilagig mit einer selbstschmierenden Messinglage ausgebildet sein.

In an sich bekannter Weise können die Schenkelteile des Beschlags nach Anspruch 11 mit dem Unterschenkel-Fassungsteil und dem Oberschenkel-Fassungsteil fest und unlösbar oder an dortigen Schienenteilen fest und lösbar angebracht sein.

Anhand einer Zeichnung wird die Erfindung näher erläutert.

Es zeigen:
- Fig. 1: eine schematische Ansicht einer Knieorthese mit bilateralen Orthesen-Beschlägen mit Knieführungsgelenken;
- Fig. 2: eine Seitenansicht eines Orthesen-Beschlags;
- Fig. 3: eine Draufsicht auf den Orthesen-Beschlag nach Fig. 2;
- Fig. 4: eine Seitenansicht von der gegenüberliegenden Seite auf den Orthesen-Beschlag nach Fig. 2;
- Fig. 5: eine Ansicht entsprechend Fig. 4 mit eingeschwenkten Schenkelteilen;
- Fig. 6: eine vergrößerte perspektivische Ansicht des Knieführungsgelenks aus einer Richtung entsprechend Fig. 4;
- Fig. 7: eine vergrößerte perspektivische Ansicht des Knieführungsgelenks aus der Richtung entsprechend Fig. 2;
- Fig. 8: eine perspektivische Ansicht des ersten Schenkelteils mit dem ersten Winkelhebelteil;
- Fig. 9: eine perspektivische Ansicht des zweiten Schenkelteils mit dem zweiten Winkelhebelteil;
- Fig. 10: eine vergrößerte perspektivische Ansicht des zweiten Scheibenteils;
- Fig. 11: eine entsprechende Ansicht des ersten Scheibenteils mit strichliert eingezeichneter Führung;
- Fig. 12: eine Darstellung der Bewegungskinematik des Knieführungsgelenks bei einer Aufschwenkbewegung; und
- Fig. 13: eine Darstellung entsprechend Fig. 12 bei einer Einschwenkbewegung des Knieführungsgelenks.

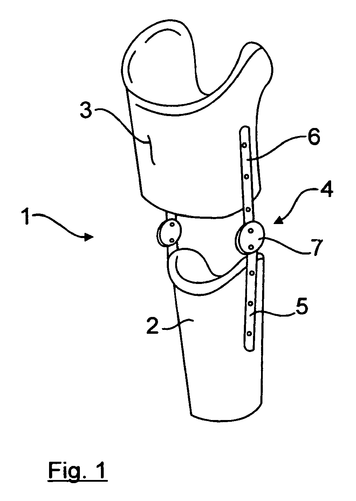

In Fig. 1 ist schematisch eine Knieorthese 1 dargestellt mit einem Unterschenkel-Fassungsteil 2 und einem Oberschenkel-Fassungsteil 3, die bilateral durch Orthesen-Beschläge 4 gelenkig verbunden sind. Jeder Orthesen-Beschlag 4 besteht aus einem unteren distalen Schenkelteil 5 und einem oberen proximalen Schenkelteil 6 aus einem Flachstahl, wobei die Schenkelteile 5, 6 mit den zugeordneten Fassungsteilen 2, 3 fest verbunden sind und untereinander eine Verbindung über ein Knieführungsgelenk 7 haben, das im Folgenden näher beschrieben werden soll.

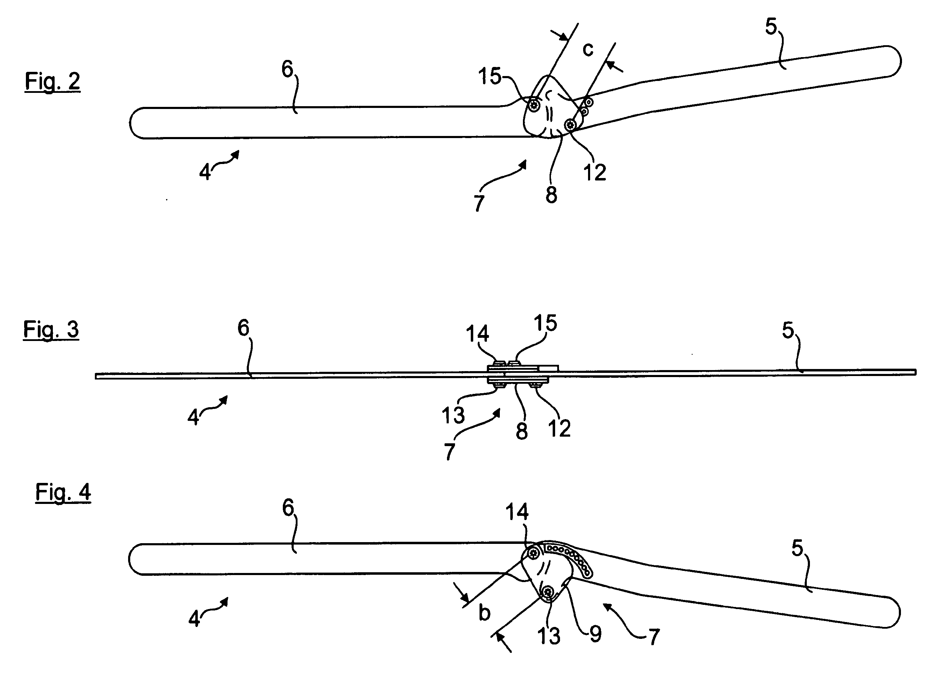

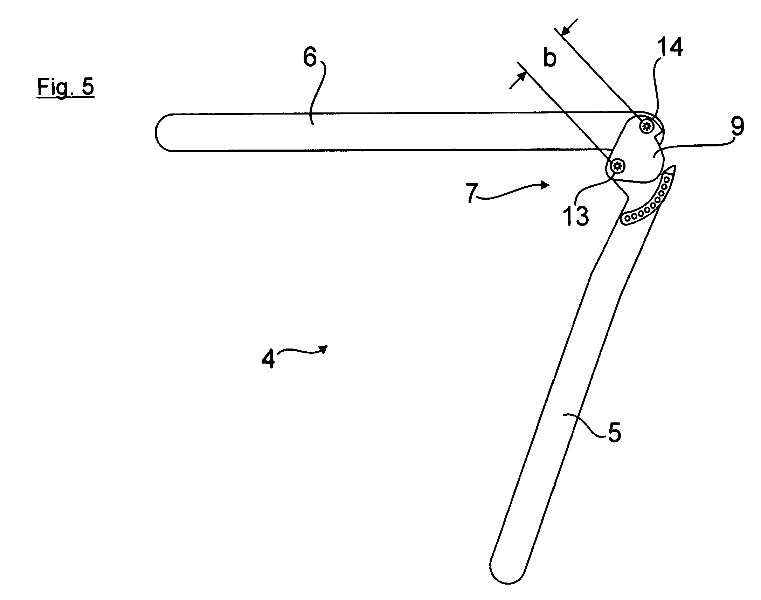

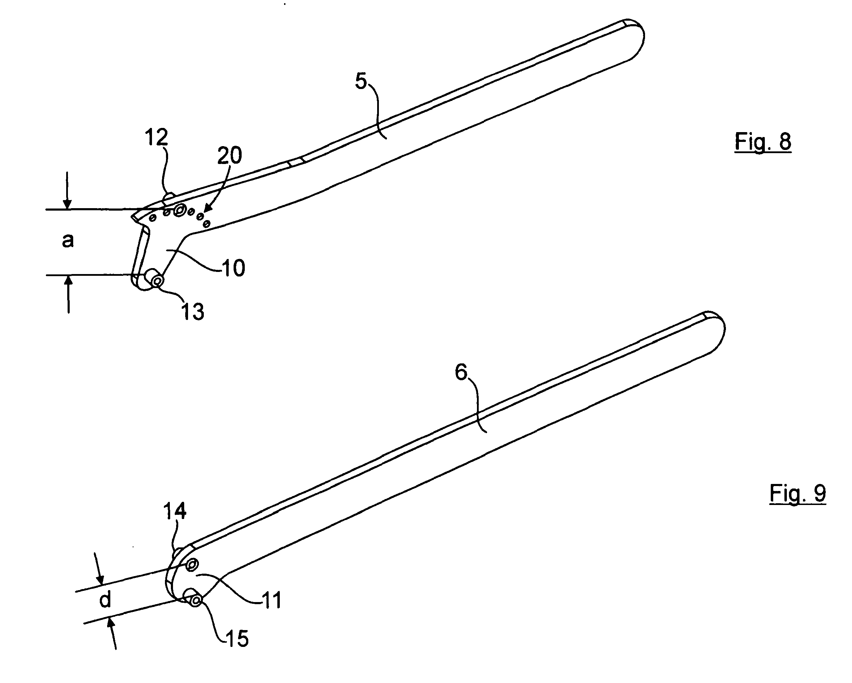

In den Fig. 2 bis 5 ist ein komplett montierter Orthesen-Beschlag 4 in unterschiedlichen Ansichten gezeigt mit jeweils einem ersten distalen Schenkelteil 5 und einem zweiten proximalen Schenkelteil 6 und dem Knieführungsgelenk 7 mit einer Draufsicht in Fig. 2 auf ein zweites Scheibenteil 8 und einer Draufsicht in den Fig. 4 und 5 auf ein erstes Scheibenteil 9. Unter den Scheibenteilen 8 und 9 ist in den Fig. 2 und 4 jeweils strichliert die stirnseitige Kontur der aneinander grenzenden Schenkelteile 5, 6 eingezeichnet. Genauer ist dies aus den Fig. 8 und 9 ersichtlich, in denen das erste distale Schenkelteil 5 und das zweite proximale Schenkelteil 6 als Einzelteile dargestellt sind:

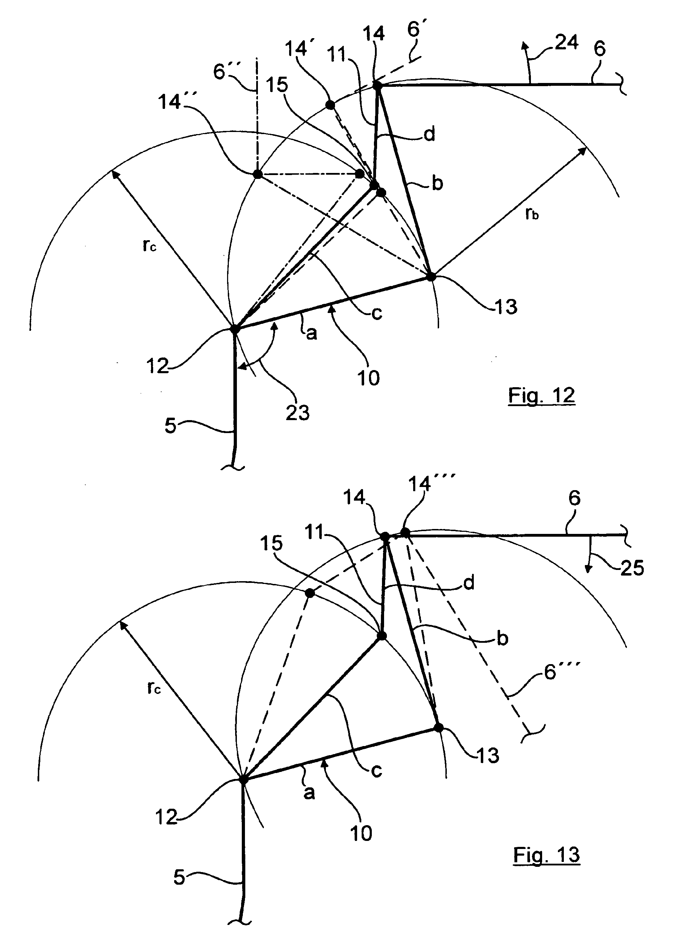

Am ersten Schenkelteil 5 ist das gelenkseitige Schenkelende mit einem ersten Winkelhebelteil 10 und das zweite proximale Schenkelteil 6 mit einem zweiten Winkelhebelteil 11 ausgebildet, wobei die Winkelhebelfunktion im Wesentlichen jeweils durch beabstandete Schwenklagerelemente 12, 13 am ersten Winkelhebelteil 10 und Schwenklagerelemente 14, 15 am zweiten Winkelhebelteil 11 gebildet sind. Mit den entsprechenden Bezugszeichen 12 bis 15 sind in allen Figuren die zugeordneten Lagerbohrungen an den Scheibenteilen 8, 9 ebenso wie die Schwenklagerstellen (in den Fig. 12 und 13) bezeichnet.

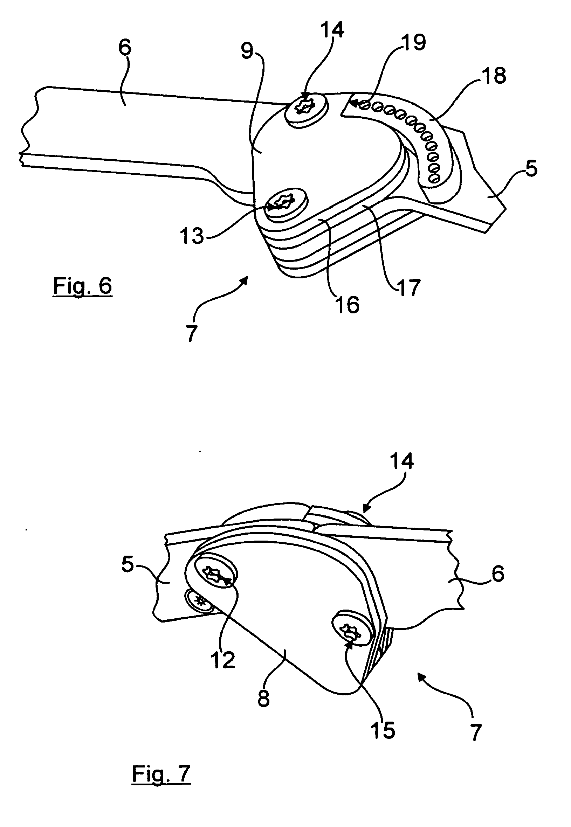

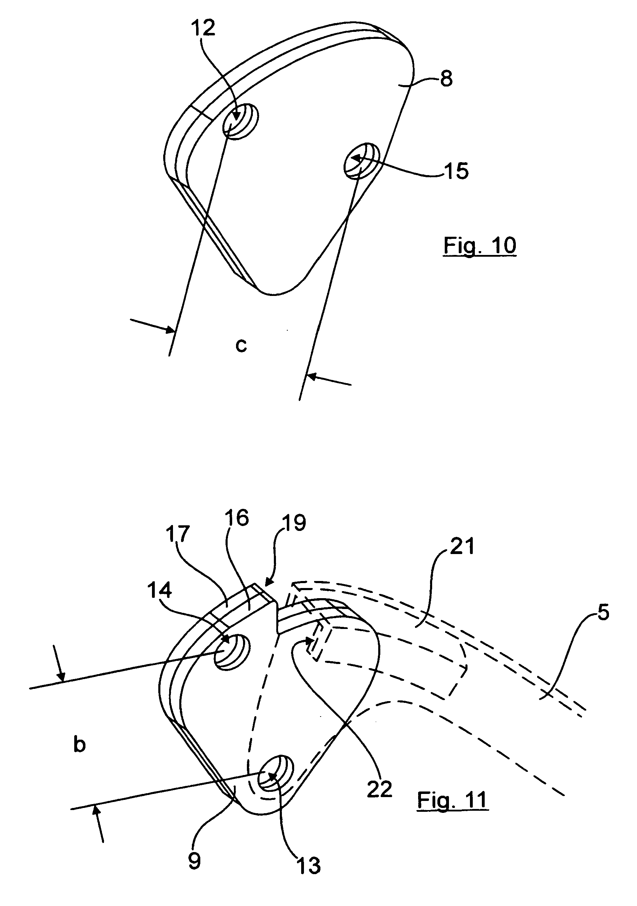

Die Schwenklagerelemente 12 bis 15 an den Schenkelteilen 5, 6 (siehe Fig. 8, 9) sind als abstehende Lagerbuchsen ausgebildet, die durch entsprechend zugeordnete Bohrungen in den Scheibenteilen 8, 9 (siehe Fig. 10, 11) gesteckt werden. Die Lagerbuchsenlängen entsprechen den Scheibenteildicken mit geringem Übermaß. In den Lagerbuchsen sind axiale Schraubenbohrungen enthalten zur Aufnahme von Schrauben, die mit Schraubenköpfe die Bohrungen der Scheibenteile 8, 9 überdecken und von außen her anliegen (siehe Fig. 6, 7).

Dabei liegen die Scheibenteile 8, 9 jeweils von außen an den Winkelhebelteilen 10, 11 an und bilden die Schwenkelemente für die dargestellte Vier-Gelenk-Anordnung. Die Scheibenteile 8, 9 sind dabei jeweils zweilagig ausgeführt mit einer äußeren stabilen Edelstahllage 16 und einer inneren Messinglage 17, wie dies beispielhaft mit den Bezugszeichen 16, 17 in den Fig. 6, 11 eingezeichnet ist. Die beiden Lagen sind dabei verklebt.

Am ersten Schenkelteil 5 ist im schwenklagerseitigen Bereich ein Anschlagbügel 18 angebracht, der mit einem zugeordneten Anschlagabsatz 19 am ersten Scheibenteil 9 zur Streckbegrenzung des Beschlags zusammenwirkt. Der Anschlagbügel 18 kann hier an einer zugeordneten Bohrungsreihe 20 am ersten Schenkelteil 5 bedarfsweise versetzt werden (Fig. 8). Alternativ zum Anschlagbügel 18 ist in Fig. 11 strichliert ein Anschlag- und Führungskanal 21 seitlich am ersten Schenkelteil 5 fest angebracht, der einerseits mit dem Anschlagabsatz 19 in der Anschlagfunktion zusammenwirkt und andererseits eine randseitige Führung für das Scheibenteil 9 ausbildet. Auch hier ist die innere Führungswandfläche des Kanals mit einer Messinglage 22 ausgerüstet.

Anhand der Fig. 12 und 13 werden die Funktion und der Bewegungsablauf des Knieführungsgelenks erläutert, die durch die Zwangsbewegung der Vier-Gelenk-Anordnung mit den Schwenklagern 12 bis 15 bestimmt sind. Wie insbesondere aus Fig. 12 ersichtlich ist, ist der Abstand der Schwenklager 12, 13 am ersten Winkelhebelteil 10 des Schwenkteils 5, als Abstand a bezeichnet, gleich mit dem Abstand b der Lager 13, 14 am ersten Scheibenteil 9 und mit dem Abstand c der Schwenklager 12, 15 am zweiten Scheibenteil 8. Der Abstand d der beiden Schwenklager 14, 15 am zweiten Winkelhebelteil 11 des zweiten Schenkelteils 6 ist dagegen nur halb so groß. In einer konkreten Ausführungsform sind dabei die Abstände a, b, c 26mm und der Abstand d 13mm. Ersichtlich sind dazu die Figuren 12 und 13 im Maßstab 2:1 gezeichnet.

Die Verbindungslinie zwischen den Schwenklagern 14, 15 am zweiten Winkelhebelteil verläuft senkrecht zur Schenkelrichtung des zweiten Schenkelteils 6. Die Verbindungslinie zwischen den Schwenklagern 12, 13 am ersten Winkelhebelteil 10 des ersten Schenkelteils 5 verläuft dagegen in einem größeren Winkel 23 von ca. 105° zur Schenkelrichtung des ersten Schenkelteils 5.

In den Fig. 12 und 13 ist jeweils mit einer durchgezogenen dicken Linie der Ausgangszustand der Vier-Gelenk-Anordnung schematisch dargestellt, wenn die beiden Schenkelteile 5, 6 in einem rechten Winkel zueinander liegen.

Entsprechend dem Pfeil 24 soll nun in der Fig. 12 der Schenkelteil 6 unter Beibehaltung der Lage des Schenkelteils 5 nach oben in eine gestrecktere Lage verschwenkt werden. Dabei bewegt sich zwangsläufig das Schwenklager 15 auf dem eingezeichneten Kreisbogen mit dem Radius rc und das Schwenklager 14 bewegt sich zwangsläufig entsprechend auf den Kreisbogen mit dem Radius rb. Die Lage auf diesen Kreisbögen ist durch die Kopplung über die Scheibenteile 8, 9 bestimmt in Verbindung mit dem Lagerabstand d.

In Fig. 12 ist eine etwas nach oben geschwenkte Lage 6' des Schenkelteils 6 strichliert eingezeichnet, wobei ersichtlich das Schwenklager 14 an die Position 14' nach vorne gewandert ist. Bei einer weiteren Streckung des Schenkelteils in die strich-punktiert gezeichnete Lage 6" hat sich das zugeordnete Schwenklager 14 noch weiter nach vorne und etwas nach unten in die Position 14" verlagert.

In Fig. 13 ist entsprechend dem Pfeil 25 bei festgehaltener Lage des Schenkelteils 5 das Schenkelteil 6 in die strichliert gezeichnete Lage 6'" nach unten eingeschwenkt, wodurch sich das Schwenklager 14 auf dem Kreisbogen mit dem Radius rb auf etwa gleicher Höhe etwas nach hinten in die Position 14"' bewegt hat.

Mit der vorstehend erläuterten Schwenkgeometrie ergibt sich eine weitgehende Nachahmung einer natürlichen Kniegelenkbewegung.

## Patentansprüche

1. Orthesen-Beschlag mit einem Knieführungsgelenk, wobei mit dem Orthesen-Beschlag (4) unilateral oder mit zwei gegenüberliegenden Orthesen-Beschlägen bilateral ein Unterschenkel-Fassungsteil (2) und ein Oberschenkel-Fassungsteil (3) der Orthese (1) gelenkig miteinander als Kniegelenkstütze verbindbar sind, wobei der Orthesen-Beschlag (4) zwei jeweils mit dem Unterschenkel-Fassungsteil (2) und dem Oberschenkel-Fassungsteil (3) fest verbindbare Schenkelteile (5, 6) aufweist, deren aufeinander zugerichtete Schenkelenden Bestandteile des Knieführungsgelenkes (7) sind, wobei das Knieführungsgelenk (7) als Vier-Gelenk-Anordnung mit vier achsparallelen Gelenken (12 bis 15) ausgebildet ist, dergestalt,
dass an den aufeinander zugenichteten Schenkelenden jeweils Winkelhebelteile (10, 11) mit jeweils zwei beabstandeten Schwenklagern (12, 13; 14, 15) ausgebildet sind, und
dass jeweils das schenkelnähere Schwenklager (12; 14) eines Winkelhebelteils (5; 6) mit dem schenketferneren Schwenklager (13; 15) des anderen Winkelhebelteils (5; 6) jeweils durch ein Schwenkelement (8, 9) verbunden sind,
**dadurch gekennzeichnet,** dass an einem ersten Winkelhebelteil (10) eines ersten Schenkelteils (5) der Abstand (a) der dortigen beiden Schwenklager (12, 13) dem Abstand (b) der beiden Schwenklager (13, 14) zwischen dem schenkelfernen Gelenklager (13) am ersten Winkelhebelteil (10) und dem schenkelnahen Schwenklager (14) des zweiten Winkelhebelteils (11) des zweiten Schenkelteils (6) und damit dem entsprechenden Abstand (b) der Schwenklager (13, 14) an einem ersten Schwenkelement (9) entspricht,
dass zudem der Abstand (a) der beiden Schwenklager (12, 13) am ersten Winkelhebelteil dem Abstand (c) der beiden Schwenklager (12, 15) zwischen dem schenkelfernen Schwenklager (15) am zweiten Winkelhebelteil (11) und dem schenkelnahen Schwenklager (12) am ersten Winkelhebelteil (10) und damit dem entsprechenden Abstand (c) der Schwenklager (12, 15) am zweiten Schwenkelement (8) entspricht, und
dass der Abstand (d) der beiden Schwenklager (14, 15) am zweiten Winkelhebelteil (11) der Hälfte des Abstands (a) der beiden Schwenklager (12,13) am ersten Winkelhebelteil (10) entspricht.

2. Orthesen-Beschlag nach Anspruch 1, **dadurch gekennzeichnet,** dass die Verbindungslinie zwischen den Schwenklagern (14, 15) am zweiten Winkelhebelteil (11) senkrecht zur Schenkelrichtung des zweiten Schenkelteils (6) verläuft, und
dass die Verbindungslinie zwischen den Schwenklagern (12, 13) am ersten Winkelhebelteil in einem Winkel (23) größer 90° zur Schenkelrichtung des ersten Schenkelteils (5) verläuft.

3. Orthesen-Beschlag nach Anspruch 1 oder 2, **dadurch gekennzeichnet,** dass die Abstände a, b, c gleich sind mit einer Abmessung von 22mm bis 30mm und entsprechend der Abstand d zugeordnet 11 mm bis 15mm ist.

4. Orthesen-Beschlag nach einem der Ansprüche 1 bis 3, **dadurch gekennzeichnet,**
dass die Schenkelteile (5, 6) einschließlich der Winkelhebelteile (10, 11) aus einem Flachmaterial, vorzugsweise aus einem Edelstahlmaterial hergestellt sind,
dass die Schwenkelemente Scheibenteile (8, 9) sind, die die flachen gegeneinander gerichteten Winkelhebelteile (10, 11) beidseitig anliegend überdecken,
dass das erste Scheibenteil (9) von einer Außenseite her am schenkelferneren Schwenklager (13) des ersten Winkelhebelteils (10) und am schenkelnäheren Schwenklager (14) des zweiten Winkelhebelteils (11) schwenkbar angeschlossen ist, und
dass das zweite Scheibenteil (8) von der gegenüberliegenden Außenseite her am schenkelferneren Schwenklager (15) des zweiten Winkelhebelteils (11) und am schenkelnäheren Schwenklager (12) des ersten Winkelhebelteils (10) schwenkbar angeschlossen ist.

5. Orthesen-Beschlag nach Anspruch 4, **dadurch gekennzeichnet,** dass die Scheibenteile (8, 9) zweilagig ausgebildet sind mit einer Außenseite aus einer Edelstahllage (16) und einer entsprechenden Innenseite aus einer Messinglage (17) und beide Lagen (16,17) verklebt sind.

6. Orthesen-Beschlag nach Ansprüche 4 oder 5, **dadurch gekennzeichnet,** dass zur Ausbildung der Schwenklager an den Winkelhebelteilen (10, 11) abstehende Lagerbuchsen angebracht sind, die formschlüssig durch zugeordnete Bohrungen in den Scheibenteilen (8, 9) gesteckt sind, wobei die Lagerbuchsenlängen den Scheibenteilendicken mit geringem Übermaß entsprechen, und dass in den Lagerbuchsen axiale Schraubenbohrungen enthalten sind zur Aufnahme von Schrauben mit Schraubenköpfen, die die Bohrungen der Scheibenteile (8, 9) überdecken und an den Scheibenteilen (8, 9) von außen her anliegen.

7. Orthesen-Beschlag nach einem der Ansprüche 1 bis 6, **dadurch gekennzeichnet,** dass an wenigstens einem der Schenkelteile (5, 6) und an einem Schwenkelement, insbesondere einem Scheibenteil (8, 9) zugeordnete, die Schenkel-Schwenkbewegung begrenzende zusammenwirkende Anschläge (18, 19; 21) angebracht sind.

8. Orthesen-Beschlag nach Anspruch 7, **dadurch gekennzeichnet,** dass am ersten Schenkelteil seitlich ein Anschlag angebracht ist, der bei einer Schenkelverschwenkung in eine gestrecktere Lage randseitig am ersten Scheibenteil (9) entlang bis zu einem dortigen, die Schwenkbewegung begrenzenden Anschlag (19) bewegbar ist.

9. Orthesen-Beschlag nach einem der Ansprüche 4 bis 8, **dadurch gekennzeichnet,** dass am ersten Schenkelteil (5) seitlich eine kanalförmige Führungsnut (21), ist, in der bei einer Schenkelverschwenkung ein Scheibenrand des ersten Scheibenteils (9) geführt ist.

10. Orthesen-Beschlag nach Anspruch 9, **dadurch gekennzeichnet,** dass die zur Scheibenteilfläche hin gerichtete und dort anliegende Führungswandfläche zweilagig ausgeführt und mit einer Messinglage (22) versehen ist.

11. Orthesen-Beschlag nach einem der Ansprüche 1 bis 10, **dadurch gekennzeichnet,** dass die Schenkelteile (5, 6) des Orthesen-Beschlags (4) mit dem Unterschenkel-Fassungsteil (2) und dem Oberschenkel-Fassungsteil (3) fest und unlösbar oder an dortigen Schienenteilen fest und lösbar angebracht sind.

## Claims

1. Orthotic fitting having a knee guide joint, wherein a lower leg mounting part (2) and an upper leg mounting part (3) of the orthosis (1) are able to be connected in a supple manner to each other as knee joint supports, either unilaterally with the orthotic fitting (4) or bilaterally with two opposing orthotic fittings, wherein the orthotic fitting (4) has two leg parts (5, 6) that can be rigidly connected to the respective lower leg mounting part (2) and the respective upper leg mounting part (3), the leg ends of which leg parts (5, 6) being components of the knee guide joint (7), said leg ends being bent towards each other, wherein the knee guide joint (7) is designed as a four-joint arrangement having four joints (12 to 15) that are axially parallel, in such a way that,
at the leg ends, which are bent towards each other, respective angled lever parts (10, 11) are formed, each having two swivel bearings (12, 13; 14, 15) that are arranged at a distance from each other, and
in each case, the swivel bearing (12; 14) that is of an angled lever part (5; 6) which is closer to the leg is connected to the swivel bearing (13; 15) of the other angled lever part (5; 6) which is further from the leg, in each case by a swivel element (8, 9),
**characterised in that,** on a first angled lever part (10) of a first leg part (5), the distance (a) of both local swivel bearings (12, 13) corresponds to the distance (b) of both swivel bearings (13, 14) between the swivel bearing (13) on the first angled lever part (10) which is further from the leg and the swivel bearing (14) of the second angled lever part (11) of the second leg part (6) which is closer to the leg, and thus to the corresponding distance (b) of the swivel bearings (13, 14) on a first swivel element (9),
that in addition, the distance (a) of both swivel bearings (12, 13) on the first angled lever part corresponds to the distance (c) of both swivel bearings (12, 15) between the swivel bearing (15) on the second angled lever part (11) which is further from the leg and the swivel bearing (12) on the first angled lever part (10) which is closer to the leg, and thus to the corresponding distance (c) of the swivel bearings (12, 15) on the second swivel element (8), and
that the distance (d) of both swivel bearings (14, 15) on the second angled lever part (11) corresponds to half the distance (a) of both swivel bearings (12, 13) on the first angled lever part (10).

2. Orthotic fitting according to claim 1, **characterised in that** the connecting line between the swivel bearings (14, 15) on the second angled lever part (11) runs perpendicular to the leg direction of the second leg part (6), and
that the connecting line between the swivel bearings (12, 13) on the first angled lever part runs at an angle (23) that is greater than 90° relative to the leg direction of the first leg part (5).

3. Orthotic fitting according to claim 1 or 2, **characterised in that** the distances a, b, c are equal to a dimension of 22mm to 30mm and, accordingly, the distance d is allocated to be 11 mm to 15mm.

4. Orthotic fitting according to one of claims 1 to 3, **characterised in that,**
the leg parts (5, 6), including the angled lever parts (10, 11), are produced from a flat material, preferably from a stainless steel material,
that the swivel elements are disc parts (8, 9) which closely cover the flat angled lever parts (10, 11), which face towards each other, on both sides,
that the first disc part (9) is rotatably connected from an outer side to the swivel bearing (13) of the first angled lever part (10) which is further from the leg and to the swivel bearing (14) of the second angled lever part (11) which is closer to the leg, and
that the second disc part (8) is rotatably connected from the opposite outer side to the swivel bearing (15) of the second angled lever part (11) which is further from the leg and to the swivel bearing (12) of the first angled lever part (10) which is closer to the leg.

5. Orthotic fitting according to claim 4, **characterised in that** the disc parts (8, 9) are formed in two layers with an outer side made from a stainless steel layer (16) and a corresponding inner side made from a brass layer (17), and both layers (16, 17) are adhered.

6. Orthotic fitting according to claim 4 or 5, **characterised in that,** for the formation of the swivel bearings, protruding bearing bushes are attached to the angled lever parts (10, 11), said bushes being positively inserted into the disc parts (8, 9) through allocated bore holes, wherein the lengths of the bearing bushes correspond to the thicknesses of the disc parts with a small excess, and that axial screw holes are contained in the bearing bushes for receiving screws having screw heads that cover the bore holes of the disc parts (8, 9) and adjoin the disc parts (8, 9) from the outside.

7. Orthotic fitting according to one of claims 1 to 6, **characterised in that** interacting stops (18, 19; 21) limiting the leg swivelling movement are attached to at least one of the swivel parts (5, 6) and to a swivel element, in particular allocated to a disc part (8, 9).

8. Orthotic fitting according to claim 7, **characterised in that** a stop is attached laterally to a first swivel part, said stop being able to be moved, while the leg is swivelling, into a stretched position along the edge of the first disc part (9) up to a local stop (19) which limits the swivelling movement.

9. Orthotic fitting according to one of claims 4 to 8, **characterised in that** a channel-shaped guide groove (21) is applied laterally to the first leg part (5), in which, while the leg is swivelling, a disc edge of the first disc part (9) is guided.

10. Orthotic fitting according to claim 9, **characterised in that** the guide wall surface pointing towards the disc part surface and adjoining it there is designed in two layers and is provided with a brass layer (22).

11. Orthotic fitting according to one of claims 1 to 10, **characterised in that** the leg parts (5, 6) of the orthotic fitting (4) are attached rigidly and non-releasably to the lower leg mounting part (2) and the upper leg mounting part (3) or are attached rigidly and releasably to local disc parts.

## Revendications

1. Armature d'orthèse comprenant une articulation de guidage de genou, telle qu'une partie de support de partie inférieure de cuisse (2) et une partie de support de partie supérieure de cuisse (3) de l'orthèse (1) peuvent être articulées l'une à l'autre en tant que dispositif de soutien de l'articulation du genou de manière unilatérale à l'aide de l'armature d'orthèse (4) et de manière bilatérale à l'aide de deux armatures d'orthèse se faisant face, sachant que l'armature d'orthèse (4) présente deux parties de cuisse (5, 6) pouvant être reliées de manière solidaire respectivement à la partie de support de partie inférieure de cuisse (2) et à la partie de support de partie supérieure de cuisse (3), dont les extrémités de cuisse orientées les unes vers les autres font partie intégrante de l'articulation de guidage du genou (7), telle que l'articulation de guidage du genou (7) est réalisée sous la forme d'un ensemble à quatre articulations pourvu de quatre articulations (12 à 15) axialement parallèles de telle manière
que respectivement des parties de levier coudées (10, 11) pourvues de respectivement deux paliers de pivotement (12, 13 ; 14, 15) espacés sont réalisées au niveau des extrémités de cuisse orientées les unes vers les autres, et
que respectivement le palier de pivotement (12 ; 14) le plus près de la cuisse d'une partie de levier coudée (5 ; 6) est relié respectivement par un élément de pivotement (8, 9) au palier de pivotement (13 ; 15), le plus éloigné de la cuisse, de l'autre partie du levier coudée (5 ; 6),
**caractérisée en ce que** la distance (a) entre les deux paliers de pivotement (12, 13) correspond, au niveau d'une première partie de levier coudée (10) d'une première partie de cuisse (5) à la distance (b) entre les deux paliers de pivotement (13, 14) entre le palier articulé (13) éloigné de la cuisse au niveau de la partie de levier coudée (10) et le palier de pivotement (14), proche de la cuisse, de la deuxième partie de levier coudée (11) de la deuxième partie de cuisse (6), et ce faisant à la distance correspondante (b) entre les paliers de pivotement (13, 14) au niveau d'un premier élément de pivotement (9),
**en ce qu'**en outre la distance (a) entre les deux paliers de pivotement (12, 13) correspond, au niveau de la première partie de levier coudée, à la distance (c) entre les deux paliers de pivotement (12, 15), entre le palier de pivotement (15) éloigné de la cuisse au niveau de la deuxième partie de levier coudée (11) et le palier de pivotement (12) proche de la cuisse au niveau de la première partie de levier coudée (10), et ce faisant à la distance (c) correspondante entre les paliers de pivotement (12, 15) au niveau du deuxième élément de pivotement (8), et
**en ce que** la distance (d) entre les deux paliers de pivotement (14, 15) au niveau de la deuxième partie de levier coudée (11) correspond à la moitié de la distance (a) entre les deux paliers de pivotement (12, 13) au niveau de la première partie de levier coudée (10).

2. Armature d'orthèse selon la revendication 1, **caractérisée en ce que** la ligne de liaison entre les paliers de pivotement (14, 15) s'étend, au niveau de la deuxième partie de levier coudée (11), perpendiculairement au sens de la cuisse de la deuxième partie de cuisse (6), et
**en ce que** la ligne de liaison entre les paliers de pivotement (12, 13) s'étend, au niveau de la première partie de levier coudée, selon un angle (23) supérieur à 90° par rapport au sens de cuisse de la première partie de cuisse (5).

3. Armature d'orthèse selon la revendication 1 ou 2, **caractérisée en ce que** les distances a, b, c sont égales et présentent une dimension allant de 22 mm à 30 mm, et **en ce que** de manière correspondante la distance d va de 11 mm à 15 mm de manière associée.

4. Armature d'orthèse selon l'une quelconque des revendications 1 à 3, **caractérisée** en ce
que les parties de cuisse (5, 6) y compris les parties de levier coudées (10, 11), sont fabriquées à partir d'un matériau plat, de préférence à partir d'un matériau en acier inoxydable,
en ce que les éléments de pivotement sont des parties de disque (8, 9) qui recouvrent, en étant contiguës au niveau des deux côtés, les parties de levier coudées (10, 11) plates orientées dans des sens opposés,
en ce que la première partie de disque (9) est raccordée de manière à pouvoir pivoter, depuis un côté extérieur, au niveau du palier de pivotement (13), le plus éloigné de la cuisse, de la première partie de levier coudée (10) et au niveau du palier de pivotement (14), le plus proche de la cuisse, de la deuxième partie de levier coudée (11), et
en ce que la deuxième partie de disque (8) est raccordée de manière à pouvoir pivoter, depuis le côté extérieur faisant face, au niveau du palier de pivotement (15) le plus éloigné de la cuisse, à la deuxième partie de levier coudée (11) et au niveau du palier de pivotement (12), le plus proche de la cuisse, à la première partie de levier coudée (10).

5. Armature d'orthèse selon la revendication 4, **caractérisée en ce que** les parties de disque (8, 9) sont formées de deux couches, et **en ce que** les deux couches (16, 17) sont collées à un côté extérieur constitué d'une couche en acier inoxydable (16) et à un côté intérieur correspondant constitué d'une couche de laiton (17).

6. Armature d'orthèse selon la revendication 4 ou 5, **caractérisée en ce que** des coussinets faisant saillie au niveau des parties de levier coudées (10, 11) sont installés pour former les paliers de pivotement, lesquels sont placés dans les parties de disque (8, 9) en passant par des alésages associés par complémentarité de forme, sachant que les longueurs des coussinets correspondent aux épaisseurs des parties de disque présentant une surdimension minime, et **en ce que** sont compris, dans les coussinets, des alésages de vissage axiaux servant à recevoir des vis pourvues de têtes de vis, qui recouvrent les alésages des parties de disque (8, 9)et qui reposent au niveau des parties de disque (8, 9) depuis l'extérieur.

7. Armature d'orthèse selon l'une quelconque des revendications 1 à 6, **caractérisée en ce que** des butées (18, 19 ; 21) associées, coopérant et délimitant le mouvement de pivotement de cuisse sont installées au niveau d'au moins une des parties de cuisse (5, 6) et au niveau d'un élément de pivotement, en particulier d'une partie de disque (8, 9).

8. Armature d'orthèse selon la revendication 7, **caractérisée en ce qu'**une butée est installée latéralement au niveau de la première partie de cuisse, laquelle butée peut être déplacée, lors d'un pivotement de cuisse, dans une position allongée, latéralement, le long de la première partie de disque (9) jusqu'à une butée (19) délimitant localement le mouvement de pivotement.

9. Armature d'orthèse selon l'une quelconque des revendications 4 à 8, **caractérisée en ce qu'**une rainure de guidage (21) en forme de canal se trouve latéralement au niveau de la première partie de cuisse (5), un bord de disque de la première partie de disque (9) étant guidé dans ladite rainure de guidage lors d'un pivotement de cuisse.

10. Armature d'orthèse selon la revendication 9, **caractérisée en ce que** la surface de paroi de guidage orientée en direction de la face de partie de disque et reposant à cet endroit est réalisée en deux couches et est pourvue d'une couche de laiton (22).

11. Armature d'orthèse selon l'une quelconque des revendications 1 à 10, **caractérisée en ce que** les parties de cuisse (5, 6) de l'armature d'orthèse (4) pourvue de la partie de support de partie inférieure de cuisse (2) et la partie de support de partie supérieure de cuisse (3) sont solidaires et inamovible ou bien sont solidaires et amovible au niveau de parties de coulisse locales.
